# EUROPÄISCHE PATENTANMELDUNG

(11) **EP 1 281 706 A1**
(43) Veröffentlichungstag der Anmeldung: **05.02.2003**
(21) Anmeldenummer: 02016266.5
(22) Anmeldetag: 23.07.2002
(51) Int. Cl.: C07D 301/32, C07D 303/04

(54) **Verfahren zur Herstellung von Alkenoxiden aus Alkenen**

(30) Priorität: 02.08.2001 DE 10137784
(71) Anmelder: BAYER AG, 51368 Leverkusen (DE)
(72) Erfinder: Weisbeck, Markus, Dr., 51067 Köln (DE); Zurlo, Christoph, Dr., 41359 Dormagen (DE); Wegener, Gerhard, Dr., 40822 Mettmann (DE); Schümmer, Günter, Dr., 50171 Kerpen (DE); Wiessmeier, Georg, Dr., 51467 Bergisch Gladbach (DE)

(57) **Zusammenfassung**

Die Erfindung betrifft ein Verfahren zur katalytischen partiellen Oxidation von Kohlenwasserstoffen in Gegenwart von Sauerstoff und wenigstens einem Reduktionsmittel, dadurch gekennzeichnet, dass man das Reaktionsgemisch durch eine Katalysator enthaltende Schicht und eine nachgeschaltete Adsorptionsmittel enthaltende Schicht, in der der partiell oxidierte Kohlenwasserstoff adsorbiert wird, leitet

## Beschreibung

Die Erfindung betrifft ein Verfahren zur katalytischen partiellen Oxidation von Kohlenwasserstoffen in Gegenwart von Sauerstoff und wenigstens einem Reduktionsmittel, dadurch gekennzeichnet, dass man das Reaktionsgemisch durch eine Katalysator enthaltende Schicht und eine nachgeschaltete Adsorptionsmittel enthaltende Schicht, in der der partiell oxidierte Kohlenwasserstoff adsorbiert wird, leitet.

Die katalytische Gasphasen-Partialoxidation von Kohlenwasserstoffen in Gegenwart von molekularem Sauerstoff und einem Reduktionsmittel ist bekannt. (DE-A1-199 59 525, DE-A1-100 23 717, US-B-5 623 090, WO-98/00413-A1, WO-98/00415-A1, WO-98/00414-A1, WO-00/59632-A1, EP-A1-0827779. WO-99/43431-A1). Als Katalysatoren werden Zusammensetzungen eingesetzt die u.a. nanoskalige Goldpartikel enthalten.

Methoden zur selektiven Trennung der Partialoxidationsprodukte von den Edukten und den Nebenprodukten aus oben genannter Partialoxidation werden jedoch nicht offenbart.

Prinzipiell sind Methoden zur Reinigung von Alkenoxiden, wie beispielsweise Propenoxid, an festen Aktivkohlen bekannt.

US-B-4 692 535 offenbart beispielsweise die Abtrennung von hoch molekularen Poly(propenoxid) von Propenoxid durch Kontakt an Aktivkohlen.

US-B-4 187 287, US-B-5 352 807 und EP-A1-0 736 528 offenbaren die Abtrennung von verschiedenen organischen Verunreinigungen von Alkenoxiden, wie Propenoxid und Butenoxid, durch Behandlung mit festen Aktivkohlen.

Eine selektive Adsorption von Partialoxidationsprodukten aus katalytischen Gasphasen-Direkt-Oxidationsreaktionen mit molekularem Sauerstoff und einem Reduktionsmittel wird aber nicht beschrieben.

Die Partialoxidation mit einem Sauerstoff-Wasserstoff-Gemisch arbeitet in einem Temperaturbereich von 140 bis 210°C und liegt damit deutlich niedriger, als Partialoxidation, bei denen nur Sauerstoff, also kein zusätzlicher Wasserstoff, verwendet wird (z.B. Ethen-Partialoxidation zu Ethenoxid; T = 210-240°C).

Die niedrige Reaktionstemperatur von << 210°C in dem erfindungsgemäßen Verfahren mit Sauerstoff und Wasserstoff hat zur Folge, dass nahezu keine Totaloxidation stattfindet und daher nur Spuren von Kohlendioxid gebildet werden. Anstelle von Kohlendioxid weist das Produktspektrum neben dem Epoxid als Hauptprodukt aber viele weitere Partialoxidationsprodukte wie Aldehyde, Ketone, Säuren, Ester, Ether in geringen Konzentrationen auf. Diese Nebenprodukte können in wässrigen Systemen den pH-Wert senken und somit die Stabilität des Epoxides verringern.

Die selektive Abtrennung von Partialoxidationsprodukten von nicht-kondensierbaren Eduktgasen - wie Kohlenwasserstoff, Sauerstoff, Wasserstoff, Verdünnungsgas - Wasser, Wasserdampf und vor allem sauer reagierenden Nebenprodukten, wie Carbonsäuren und Aldehyden, ist nicht offenbart.

Alle publizierten Anmeldungen zur selektiven Oxidation von Kohlenwasserstoffen in Gegenwart von Sauerstoff und einem Reduktionsmittel erreichen nur einen kleinen Kohlenwasserstoffumsatz von kleiner 10 %. Alle Verfahren arbeiten daher bei einer technischen Realisierung mit sehr großen Kreislaufgasmengen. Die Isolierung von sehr kleinen Volumina an Wertprodukten (z.B. 2 Vol.-% Kohlenwasserstoffoxid) aus großen Gasmengen (z.B. 98 Vol.-% Gas bestehend aus Kohlenwasserstoff, Wasserstoff, Sauerstoff, Wasser, Acetaldehyd, Propionaldehyd, Aceton, Essigsäure, Formaldehyd) ist sehr aufwendig. Die Wirtschaftlichkeit der beschriebenen selektiven Oxidationen wird daher entscheidend von den Kosten der Wertproduktisolierung bestimmt.

Die Aufgabe der vorliegenden Erfindung ist die Bereitstellung eines Abtrenn-Verfahrens für das Produkt aus dem Reaktionsgemisch bei der Herstellung von Kohlenwasserstoffoxiden durch partielle Oxidation von Kohlenwasserstoff in Gegenwart von Sauerstoff und eines Reduziermittels und eines Katalysators.

Eine weitere Aufgabe der vorliegenden Erfindung ist die Bereitstellung eines Verfahrens, bei dem ein hoher Gesamtumsatz an Alken erzielt wird.

Die Aufgabe wird erfindungsgemäß gelöst durch ein Verfahren zur katalytischen partiellen Oxidation von Kohlenwasserstoffen in Gegenwart von Sauerstoff und wenigstens einem Reduktionsmittel, dadurch gekennzeichnet, dass man das Reaktionsgemisch durch eine Katalysator enthaltende Schicht und eine nachgeschaltete Adsorptionsmittel enthaltende Schicht, in der der partiell oxidierte Kohlenwasserstoff adsorbiert wird, leitet.

Unter dem Begriff Kohlenwasserstoff werden ungesättigte oder gesättigte Kohlenwasserstoffe wie Olefine oder Alkane verstanden, die auch Heteroatome wie N, O, P, S oder Halogene enthalten können. Die zu oxidierende organische Komponente kann azyklisch, monozyklisch, bizyklisch oder polyzyklisch und kann monoolefinisch, diolefinisch oder polyolefinisch sein.

Bei Kohlenwasserstoffen mit zwei oder mehreren Doppelbindungen können die Doppelbindungen konjugiert und nichtkonjugiert vorliegen. Bevorzugt werden Kohlenwasserstoffe oxidiert, aus denen solche Oxidationsprodukte gebildet werden, deren Partialdruck niedrig genug liegt, um das Produkt ständig vom Katalysator zu entfernen. Bevorzugt sind ungesättigte und gesättigte Kohlenwasserstoffe mit 2 bis 20, vorzugsweise 2 bis 12 Kohlenwasserstoffatomen, insbesondere Ethen, Ethan, Propen, Propan, Isobutan, Isobutylen, 1-Buten, 2-Buten, cis-2-Buten, trans-2-Buten, 1,3-Butadien, Pentene, Pentan, 1-Hexen, Hexene, Hexan, Hexadien, Cyclohexen, Benzol.

Der Sauerstoff kann in verschiedenster Form eingesetzt werden, z.B. molekularer Sauerstoff, Luft und Stickstoffoxid. Molekularer Sauerstoff wird bevorzugt.

Als Reduktionsmittel eignet sich insbesondere Wasserstoff. Es kann jede bekannte Wasserstoffquelle genutzt werden, wie z.B. reiner Wasserstoff, Cracker-Wasserstoff, Synthesegas oder Wasserstoff aus Dehydrierung von Kohlenwasserstoffen und Alkoholen. In einer anderen Ausführungsform der Erfindung kann der Wasserstoff auch in einem vorgeschalteten Reaktor in situ erzeugt werden, z.B. durch Dehydrierung von Propan oder Isobutan oder Alkoholen wie Isobutanol. Der Wasserstoff kann auch als Komplex-gebundene Spezies, z.B. Katalysator-Wasserstoffkomplex, in das Reaksystem eingeführt werden.

Zu den essentiell notwendigen oben beschriebenen Eduktgasen kann optional auch ein Verdünnungsgas, wie Stickstoff, Helium, Argon, Methan, Kohlendioxid, Kohlenmonoxid oder ähnliche, sich überwiegend inert verhaltende Gase, eingesetzt werden. Auch Mischungen der beschriebenen Inertkomponenten können eingesetzt werden. Der Inertkomponentenzusatz ist zum Transport der freiwerdenden Wärme dieser exothermen Oxidationsreaktion und aus sicherheitstechnischen Gesichtspunkten oft günstig. Wird der erfmdungsgemäße Prozess in der Gasphase durchgeführt, werden bevorzugt gasförmige Verdünnungskomponenten wie z.B. Stickstoff, Helium, Argon, Methan und evtl. Wasserdampf und Kohlendioxid verwendet. Wasserdampf und Kohlendioxid sind zwar nicht völlig inert, bewirken aber bei kleinen Konzentrationen (< 2 Vol.-% der gesamten Reaktionsgase) häufig einen positiven Effekt.

Das relative molare Verhältnis von Kohlenwasserstoff, Sauerstoff, Reduktionsmittel (insbesondere Wasserstoff) und optional einem Verdünnungsgas ist in weiten Bereichen variierbar.

Bevorzugt wird im Bereich von 1-30 mol-%, besonders bevorzugt 5-25 mol-% Sauerstoff eingesetzt.

Bevorzugt wird ein Überschuss von Kohlenwasserstoff, bezogen auf eingesetzten Sauerstoff (auf molarer Basis) eingesetzt. Der Kohlenwasserstoffgehalt liegt typischerweise größer 1 mol-% und kleiner als 96 mol-%. Bevorzugt werden Kohlenwasserstoffgehalte im Bereich von 5 bis 90 mol-%, besonders bevorzugt von 20 bis 85 mol-% eingesetzt. Der molare Reduktionsmittelanteil (insbesondere Wasserstoffanteil) - in Bezug auf die Gesamtmolzahl aus Kohlenwasserstoff, Sauerstoff, Reduktionsmittel und Verdünnungsgas - kann in weiten Bereichen variiert werden. Typische Reduktionsmittelgehalte liegen bei größer als 0,1 mol-%, bevorzugt bei 2-80 mol-%, besonders bevorzugt bei 3-70 mol-%.

Als Katalysatoren werden vorteilhaft Zusammensetzungen enthaltend Edelmetallpartikel mit einem Durchmesser kleiner 51 nm auf einem Titan- und/oder Siliziumanteile enthaltendem Trägermaterial eingesetzt.

Bevorzugt werden Gold und/oder Silber als Edelmetallpartikel eingesetzt. Bevorzugt besitzen die Goldpartikel einen Durchmesser im Bereich von 0,3 bis 10 nm, bevorzugt 0,9 bis 9 nm und besonders bevorzugt 1,0 bis 8 nm. Bevorzugt besitzen die Silberpartikel einen Durchmesser im Bereich von 0,5 bis 50 nm, bevorzugt 0,5 bis 20 nm und besonders bevorzugt 0,5 bis 15 nm.

Als Katalysator-Trägermaterialien werden bevorzugt die in DE-A1-199 59 525 und DE-A1-100 23 717 genannten Trägermaterialien, besonders bevorzugt organischanorganische Hybridmaterialien (Hybridträgermaterialien, Ormosile) verwendet.

Organisch-anorganische Hybridmaterialien im Sinne der Erfindung sind organisch modifizierte Gläser, die bevorzugt in Sol-Gel-Prozessen über Hydrolyse- und Kondensationsreaktionen löslicher Vorläuferverbindungen entstehen und im Netzwerk nicht hydrolysierbare terminale und/oder verbrückende organische Gruppen enthalten. Diese Materialien und deren Herstellung ist u.a. in DE-A1-199 59 525, DE-A1-100 23 717 offenbart, welche hiermit für die Zwecke der US-Patentpraxis als Referenz in die vorliegende Anmeldung aufgenommen werden.

Ganz besonders bevorzugte Hybridträgermaterialien mit einem Anteil an im Sol-Gel-Netzwerk eingebauten und/oder eingelagerten freien Silizium-Wasserstoff Einheiten lassen sich besonders vorteilhaft aus Titan- und Silanvorläuferverbindungen gemäss DE-A1-199 59 525 herstellen.

Zur Generierung von Goldpartikel auf den Trägermaterialien eignen sich die in den Dokumenten US-A-5 623 090, WO-98/00413-A1, WO-98/00415-A1, WO-98/00414-A1, WO-00/59632-A1, EP-A1-0827779 und WO-99/43431-A1 offenbarten Verfahren, wie Abscheidung-Ausfällung (Deposition-Precipitation), Coprecipitation, Imprägnierung in Lösung, Incipient-wetness, Kolloid-Verfahren, Sputtern, CVD (chemical vapor deposition), PVD (physical vapor deposition) und Mikroemulsion. Die Methoden Incipient Wetness, Lösungsmittelimprägnierung und eine Kombination aus Imprägnierung der Trägermaterialien mit Edelmetallprecursorn und sofort anschließender Trocknung durch Sprüh- oder Wirbelbetttechnologie werden in den Anmeldungen DE-A1-199 59 525, DE-A1-100 23 717 offenbart und sind besonders vorteilhaft.

Die Trägermaterialien können auch Anteile von Promotor-Metallen aus der Gruppe 5 des Periodensystems nach IUPAC (1985), wie Vanadium, Niob und Tantal, bevorzugt Tantal, der Gruppe 3, bevorzugt Yttrium, der Gruppe 4, bevorzugt Zirkon, der Gruppe 8, bevorzugt Fe, der Gruppe 15, bevorzugt Antimon, der Gruppe 13, bevorzugt Aluminium, Bor, Thallium und Metalle der Gruppe 14, bevorzugt Germanium, enthalten. Die zusätzlichen Metalle (Promotoren) liegen häufig in oxidischer Form vor.

Die erfindungsgemäßen edelmetallhaltigen Zusammensetzungen können bei Temperaturen > 10°C, bevorzugt im Bereich von 80-250°C, besonders bevorzugt im Bereich von 120-215°C eingesetzt werden. Bei den hohen Temperaturen kann in gekoppelten Anlagen Dampf als Energieträger erzeugt werden. Bei geschickter Verfahrensführung kann der Dampf z.B. zur Produktaufarbeitung genutzt werden.

Vorteilhaft wird die Oxidationsreaktion bei erhöhten Reaktionsdrücken durchgeführt. Bevorzugt sind Reaktionsdrücke von > 1 bar, besonders bevorzugt 2-50 bar.

Die Katalysatorbelastung kann in weiten Bereichen variiert werden. Bevorzugt werden Katalysatorbelastungen von 0,5-100 l Gas pro ml Katalysator und Stunde verwendet, besonders bevorzugt werden Katalysatorbelastungen von 2-50 l Gas pro ml Katalysator und Stunde gewählt.

Bei der katalytischen Oxidation von Kohlenwasserstoffen in Gegenwart von Wasserstoff entsteht in der Regel Wasser als Koppelprodukt zum entsprechenden selektiven Oxidationsprodukt.

Die kontinuierliche Trennung der bei der Direkt-Oxidation in Gegenwart von Sauerstoff und einem Reduktionsmittel entstehenden Partialoxidationsprodukte vom Reaktionsgemisch gelingt überraschend auch in Gegenwart von Wasser und / oder Wasserdampf und sauer reagierenden Nebenprodukten durch selektive Adsorption an geeigneten Adsorbentien ohne Zersetzung dieser Adsorptionsprodukte.

Als Adsorptionsmittel kommen daher alle Feststoffe in Frage, die in der Lage sind, partiell oxidierte Kohlenwasserstoffe ohne Zersetzung auch in Gegenwart von Wasser und/oder Wasserdampf und sauer reagierenden Nebenprodukten zu adsorbieren. Die Adsorptionsmittel enthaltende Schicht darf dabei keine Folgereaktionen der adsorbierten Partialoxidationsprodukte initiieren.

Bevorzugt werden als Adsorptionsmittel Zeolithe, bzw. Molsiebe und Aktivkohlen verwendet. Bei Zeolithen werden hydrophobe Zeolithe mit Porengrößen im Bereich von 0,3 bis 100 nm, bevorzugt 3,1 bis 50 nm wie Wesalith DAZ F20 (Degussa AG) und Wesalith DAY F20 (Firma Degussa AG) bevorzugt. Organisch modifizierte Zeolithe, z.B. nach Silylierung oder Behandlung mit fluororganischen Materialien, können ebenfalls vorteilhaft verwendet werden.

Wasser wird an den relativ hydrophoben Zeolithen häufig nur unvollständig adsorbiert. Bei verschiedenen Verfahren, wie beispielsweise Druckwechsel- oder Temperaturwechsel-Ad(De)sorption kann es daher günstig sein, hinter der Adsorptionsmittel enthaltenden Schicht noch eine weitere für die Adsorption von Wasser geeignete Schicht, bestehend aus z.B. 3A-Molekularsieben, zu betreiben.

Nach einer gewissen Zeit muss das adsorbierte Reaktionsprodukt (Kohlenwasserstoffoxid) wieder vom Adsorptionsmittel entfernt werden.

Die Adsorption und anschließende Desorption kann hierbei nach den bekannten Verfahren wie Druckwechsel-Adsorption/Desorption, Temperaturwechsel-Adsorption/Desorption oder Desorption durch Wasserdampfbehandlung durchgeführt werden.

Die Kohlenwasserstoffoxid-Adsorption wird mit steigenden Drücken und/oder fallenden Temperaturen begünstigt, und durch Erwärmung und/oder Druckerniedrigung verringert.

Die Kohlenwasserstoffoxid-Adsorption erfolgt vorteilhaft bei Reaktionsdruck von ca. 1-30 bar. Die anschließende Kohlenwasserstoffoxid-Desorption erfolgt anschließend vorteilhaft bei verringertem Druck. Aus wirtschaftlichen Gründen muss dabei ein Kompromiss zwischen leichter Kohlenwasserstoffoxid-Desorption bei geringen Drücken und Kosten für die anschließende Gaskomprimierung gefunden werden. Vorzugsweise wird ein Druckunterschied zwischen Adsorption und Desorption von ≤30 bar, besonders bevorzugt von < 25 bar eingestellt.

Da die Adsorption durch Temperaturerhöhung verringert wird, kann adsorbiertes Kohlenwasserstoffoxid auch durch Aufheizen des beladenen Adsorberbettes desorbiert werden. Zur Erhaltung der vollständigen Kohlenwasserstoffoxid - Selektivität sollte die Temperatur im Adsorber während des Prozessschrittes "Regeneration" bei maximal 200°C, vorzugsweise maximal 180°C liegen.

Der von Partialoxidationsprodukten abgereicherte Gasstrom wird vorzugsweise zur erneuten Reaktion eventuell nach einer weiteren Reinigung, z.B. Trocknung, in den Reaktor zurückgeführt. Dieser Gasstrom besteht im wesentlichen aus nicht umgesetztem Kohlenwasserstoff, Reduktionsmittel, Sauerstoff und evtl. einem Verdünnungsgas. Durch diese Kreislauffahrweise mit regelmäßiger Abtrennung der Reaktionsprodukte können wesentlich erhöhte Gesamtumsätze erreicht werden. Die Aufkonzentrierung der Reaktionsprodukte in der Adsorptionsmittel enthaltenden Schicht senkt den Aufarbeitungsaufwand deutlich.

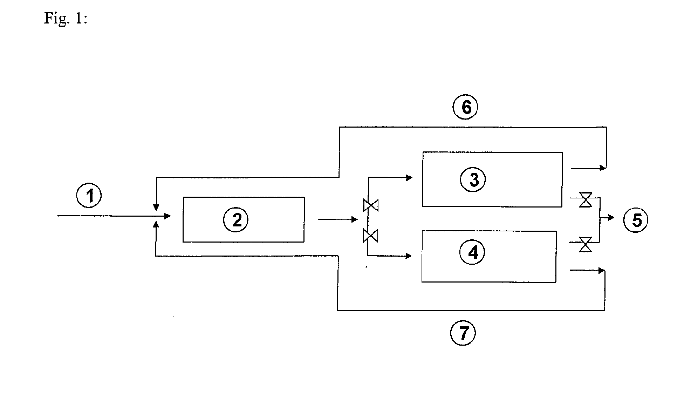

Ein Fliessbild eines Verfahrens für die Oxidation von Propylen zu Propylenoxid mit zwei Adsorbern ist in Figur 1 dargestellt. Hier steht 1 für Feed (C₃H₆, H₂, O₂), 2 für Reaktor, 3 für PO-Ad(De)sorption, 4 für PO-Ad(De)sorption, 5 für PO, 6 für C₃H₆/H₂/O₂ und 7 ebenfalls für C₃H₆/H₂/O₂.

Die Adsorptionsmittel enthaltende Schichten (Adsorber) werden, insbesondere im Falle von drei oder mehr Adsorbern, vorteilhaft im Wechsel betrieben, d.h. ein Adsorber befindet sich im Prozessschritt "Beladen", der zweite im Prozessschritt "Regeneration" und der dritte (falls vorhanden oder auch jeder weitere eventuell vorhandene Adsorber) im Prozessschritt "Stand-by". Der produkthaltige Gasstrom wird z.B. mittels eines Ventilators über den in Stellung "Beladen" befindlichen Adsorber befördert. Der produkthaltige Gasstrom durchströmt den Adsorber vorteilhaft von unten nach oben.

Beim Beladevorgang wandert die Massenübergangszone vorteilhaft in der Adsorberschicht von unten nach oben. Die Produkt-Konzentration wird vorteilhaft von einem Analysengerät (z.B. GC-Gerät) überwacht und registriert. Bei Erreichen einer Grenzkonzentration kann die Adsorption auf den Adsorber, welcher sich in Bereitschaft befindet, umgeschaltet werden. Der vorher beladene Adsorber wird in Regeneration umgeschaltet.

Die Umschaltung von "Beladen" auf "Regeneration" erfolgt beispielsweise entweder automatisch über eine FID-GC-Messung (Flammen-Ionisations-Detektor, Gas-Chromatographie-Analyse) oder innerhalb gewisser Zeitintervalle oder manuell im Handbetrieb.

Die "Regeneration" kann auf mehrere Arten erfolgen, z.B. durch Druckwechselad(de)sorption oder Temperaturwechselad(de)sorption, oder mit Wasserdampf.

Regeneration des Adsorberbettes mit Wasserdampf umfasst vorteilhaft die Arbeitsschritte Bedampfen, Spülen mit Inertgas, beispielsweise Stickstoff, Trocknen und ggf. Kühlen. Die Desorption von Kohlenwasserstoffoxid erfolgt vorzugsweise mit im Bereich von 80-150°C heißem, überhitzten Wasserdampf bei Normaldruck oder erhöhten Apparatedrücken. Die Bedampfung der Adsorber erfolgt vorteilhaft im Gegenstrom zum Kohlenwasserstoffoxid-haltigen Reaktionsgas von oben nach unten. Bei dieser Vorgehensweise wird dafür gesorgt, dass die oberste Adsorberschicht praktisch frei von Kohlenwasserstoffoxid bleibt. Durch das Bedampfen wird das Adsorberbett auf beispielsweise im Bereich von 70-150°C aufgeheizt und das adsorbierte Kohlenwasserstoffoxid desorbiert. Mit dem Dampfstrom wird das Kohlenwasserstoffoxid zum Adsorberboden gerührt, wo es den Adsorber über die Regeneratleitung verlässt.

In den Regeneratkühlern findet die Kondensation des Wasserdampf-Kohlenwasserstoffoxid-Gemisches statt.

Als Kühlmedium wird beispielsweise Kühlwasser oder Sole von beispielsweise 20°C im Gegenstrom zum Betriebsmedium verwendet.

Bevorzugt fließt das kondensierte und gekühlte Regenerat im freien Gefälle zum Sumpfbehälter und wird von dort mittels Pumpe zur Kohlenwasserstoffoxidseparierung gefördert.

Nach der Bedampfung ist die Adsorberschicht heiß und feucht. Bei einer Trocknung und Kühlung mit Luft besteht die Gefahr der Bildung explosiver Gasgemische. Der maximale Sauerstoffgehalt im System sollte daher < 20 Vol.-% betragen. Die Überwachung des Sauerstoffgehaltes erfolgt beispielsweise über ein Sauerstoffmessgerät.

Die Trocknung und Kühlung der Adsorberschicht erfolgt daher vorteilhaft mit Inertgasen wie beispielsweise Stickstoff. Aus wirtschaftlichen Gründen erfolgt die Inergasspülung im geschlossenen Kreislauf.

Dazu wird der Adsorber zunächst mit Stickstoff gespült (ca. dreifaches Adsorbervolumen). Der Stickstoff verdrängt den Wasserdampf und strippt gleichzeitig aus dem des Absorbers anhaftendem Kontaktwasser die restlichen Kohlenwasserstoffoxidmengen aus.

Das Trocknen der Absorberschicht erfolgt vorzugsweise in einem geschlossenen Rohrleitungskreislauf mittels Inertgas (z.B. Stickstoff). Mit einem Ventilator wird beispielsweise der Stickstoffgasstrom von unten nach oben über das Adsorberbett geführt. Der Stickstoffstrom wird im dampfbeheizten Wärmetauscher auf beispielsweise im Bereich von 100-130°C erwärmt und heizt die durch Wasserverdampfung zunächst auf Kühlgrenztemperatur abgekühlte Adsorberschicht auf und desorbiert hierbei adsorbiertes Wasser. Nach Verlassen des Adsorbers wird der warme, feuchte Stickstoffstrom mittels beispielsweise zweier hintereinandergeschalteter Wärmetauscher kondensiert und gekühlt. Das anfallende Wasser/Lösemittelkondensat wird in den Sumpfbehälter geleitet. Sobald die Temperatur in der Mitte des Adsorberbettes ansteigt, wird der Trocknungsvorgang beendet.

Der Kühlvorgang verläuft analog zum Trockenvorgang mit der Ausnahme, dass der Erhitzer ausgeschaltet ist. Zu Beginn des Kühlers wird die untere Schicht des Adsorberbettes gekühlt, während die obere Schicht durch den bei der Kühlung verdrängten aufgeheizten Stickstoffstrom, immer noch geheizt und getrocknet wird. Gleichzeitig werden aus der oberen Adsorberschicht weitere Lösemittel desorbiert.

Sobald der Adsorber auf beispielsweise 30°C abgekühlt ist, kann der vollständig regenerierte Adsorber wieder beladen werden.

Besonders bevorzugt wird in dem erfindungsgemäßen Verfahren Propen zu Propenoxid oxidiert.

Das erfindungsgemäße Verfahren ist auch in der Flüssigphase zur Oxidation von Kohlenwasserstoffe geeignet. Bei Ausführung der Erfindung in der Flüssigphase wird zweckmäßigerweise eine oxidationsstabile und thermisch stabile inerte Flüssigkeit gewählt, wie Alkohole, Polyalkohole, Polyether, halogenierte Kohlenwasserstoffe, Silikonöle. Sowohl in Gegenwart von organischen Hydroperoxiden (ROOH) werden z. B. Olefine in der Flüssigphase selektiv an den beschriebenen Katalysatoren zu Epoxiden umgesetzt, als auch in Gegenwart von Wasserstoffperoxid oder in Gegenwart von Sauerstoff und Wasserstoff werden Olefine in der Flüssigphase selektiv an den beschriebenen Katalysatoren zu Epoxiden umgesetzt.

Die charakteristischen Eigenschaften der vorliegenden Erfindung werden an Hand von Testreaktionen in den folgenden Beispielen veranschaulicht.

### Beispiele

### Vorschrift zum Test der Adsorption von katalytisch hergestelltem Roh-Propenoxid an geeigneten festen Adsorbern (Testvorschrift)

Es wurde ein Metallrohrreaktor mit 10 mm Innendurchmesser und 30 cm Länge eingesetzt, welcher mittels eines Ölthermostaten temperiert wurde. Der Reaktor wurde mit einem Satz von vier Massendurchflussregler (Kohlenwasserstoff, Sauerstoff, Wasserstoff, Stickstoff) mit Eduktgasen versorgt. Zur Reaktion wurden 1 g Katalysator (2x2 mm; siehe nachfolgende Katalysatorpräparationsvorschrift) bei 160°C und 3 bar vorgelegt. Die Standardkatalysatorbelastung lag bei 5 l Gas / (g Kat. x h). Als Kohlenwasserstoff wurde Propen eingesetzt. Die Katalysatorproduktivität liegt bei Verwendung von Propen als Kohlenwasserstoff bei 200 g Propenoxid/(kg Kat. x h) Der Reaktionsgasstrom wurde anschließend durch einen nachgeschalteten Adsorber (Metallrohr, 10 mm Innendurchmesser und 30 cm Länge; gefüllt mit Adsorberfestbett) unter Systemdruck geleitet. Zur Desorption wurde der beladene Adsorber auf 100 mbar entspannt und das desorbierte Material mittels einer Kühlfalle (-40°C) aufgefangen.

Zur Durchführung der Oxidationsreaktionen wurde ein Gasstrom, nachfolgend immer als Standard-Gaszusammensetzung bezeichnet, ausgewählt: C₃H₆ / H₂ / O₂ / : 60 / 30 / 10 % Vol.-%,. Die Reaktionsgase wurden gaschromatographisch vor und hinter dem Adsorber quantitativ analysiert. Die gaschromatographische Auftrennung der einzelnen Reaktionsprodukte erfolgte durch eine kombinierte FID/WLD-Methode, bei der drei Kapillarsäulen durchlaufen werden:
FID: HP-Innowax, 0,32 mm Innendurchmesser, 60 m lang, 0,25 µm Schichtdicke.
WLD: Hintereinanderschaltung von
   HP-Plot Q, 0,32 mm Innendurchmesser, 30 m lang, 20 µm Schichtdicke
   HP-Plot Molsieve 5 A, 0,32 mm Innendurchmesser, 30 m lang, 12 um Schichtdicke.
(FID = Flammen-Ionisation-Detektor; WLD = Wärme-Leitfähigkeitsdetektor)

### Katalysatorherstellung

Dieses Beispiel beschreibt zunächst die Präparation eines pulverförmigen katalytisch aktiven organisch-anorganischen Hybridmaterials, bestehend aus einem Silizium- und Titan-haltigen, organisch-anorganischen Hybridmaterial mit freien Silanwasserstoffeinheiten, welches mit Goldteilchen (0,04 Gew.-%) über Incipient-Wetness belegt wurde. Anschließend wird das feinpulverige Katalysatormaterial in Extrudate überführt.

184,29 g Methyltrimethoxysilan (1,35 mol) und 25,24 g Triethoxysilan (153,6 mmol) werden vorgelegt. 44,79 g p-Toluolsulfonsäure (0,1 n) werden hinzugefügt und anschließend mit 17,14 g Tetrapropoxytitan, gelöst in 40 g Ethanol, versetzt. Nach einer Alterungszeit von 12 h wurde das Gel zwei mal mit je 200 ml Hexan gewaschen, 2 h bei RT und 8 Stunden bei 120°C unter Luft getrocknet.

10,1 g getrocknetes Sol-Gel-Material wurde mit 5 g einer 0,16 %igen Lösung von HAuCl₄ x H₂O in Methanol unter Rühren imprägniert (Incipient Wetness), bei RT im Luftstrom getrocknet, dann 8 h bei 120°C unter Luft und anschließend 5 h bei 400°C unter Stickstoffatmosphäre getempert. Das so hergestellte katalytisch aktive organisch-anorganische Hybridmaterial enthält 0,04 Gew.-% Gold.

### Extrudatbildung

8,5 g organisch-anorganisches Hybridmaterial, synthetisiert gemäß obiger Katalysatorherstellung wurden mit 5 g Siliziumdioxidsol (Levasil, Bayer, 300 m²/g, 30 Gew.-% SiO₂ in Wasser) und 1,0 g SiO₂-Pulver (Ultrasil VN3, Degussa) 2 h lang intensiv vermischt. Die erhaltene plastische Masse wurde mit 2 g Natriumsilicatlösung (Aldrich) versetzt, 5 min intensiv homogenisiert und dann in einer Strangpresse zu 2 mm-Strängen verformt. Die so hergestellten Stränge wurden zunächst 8 h bei Raumtemperatur und dann 5 h bei 120°C getrocknet und anschließend 4 h unter Stickstoffatmosphäre bei 400°C getempert. Der mechanisch stabile Formkörper hat eine hohe Seitendruckfestigkeit.

Die getemperten 2x2 mm Formkörper wurden als Katalysator in der Gasphasen-Epoxidation von Propen mit molekularem Sauerstoff in Gegenwart von Wasserstoff verwendet.

### Beispiel 1

Das Reaktionsgas (Analyse am Reaktorausgang; vor Adsorber) enthält 1,5 Vol.-% Propenoxid, 2,5 Vol.-% Wasser, 0,15 Vol.-% Nebenprodukte (u.a. Acetaldehyd, Propionaldehyd, Aceton, Essigsäure). Das Reaktionsgas wurde durch einen Adsorber geleitet, der mit 5 g DAY F20 (Firma Degussa) gefüllt wurde. Die Propenoxid-Gaskonzentration nach dem Adsorber wurde per GC in Abhängigkeit von der Zeit gemessen. Die Kapazität von DAY F20 an Propenoxid beträgt ca. 200 g Propenoxid/(kg DAY x h).

| Zeit [h] | Propenoxid-Konzentration in der Gasphase [Vol.-%] (hinter Adsorber) |
|---|---|
| 0 | 0 |
| 1 | 0 |
| 2 | 0 |
| 2,5 | 0,1 |
| 2,7 | 0,2 |
| 3 | 0,3 |
| 4 | 1,0 |
| 5 | 1,45 |
| 6 | 1,46 |

Das adsorbierte Epoxid kann unter Druckverminderung auf 100 mbar zu 90 % desorbiert werden. Es wurden fünf Zyklen "Beladen des Adsorbens" und "Regeneration des Adsorbens" gefahren. Ab Zyklus 2 betrug die PO-Desorptionsrate > 97 %.

### Beispiel 2

Das Reaktionsgas (Analyse am Reaktorausgang; vor Adsorber) enthält 1,5 Vol.-% Propenoxid, 2,5 Vol.-% Wasser, 0,15 Vol.-% Nebenprodukte (u.a. Acetaldehyd, Propionaldehyd, Aceton, Essigsäure). Das Reaktionsgas wurde durch einen Adsorber geleitet, der mit 5 g DAZ F20 (Firma Degussa) gefüllt wurde. Die Propenoxid-Gaskonzentration nach dem Adsorber wurde per GC in Abhängigkeit der Zeit gemessen.

Die Kapazität von DAZ F20 an Propenoxid beträgt ca. 100 g Propenoxid/(kg DAZ x h).

| Zeit [h] | Propenoxid-Konzentration in der Gasphase [Vol.-%] nach dem Absorber |
|---|---|
| 0 | 0 |
| 1 | 0 |
| 2 | 0,4 |
| 2,5 | 1,4 |
| 3 | 1,46 |

Das adsorbierte Epoxid kann unter Druckverminderung auf 100 mbar zu 84 % desorbiert werden. Es wurden fünf Zyklen "Beladen des Adsorbens" und "Regeneration des Adsorbens" gefahren. Ab Zyklus 2 betrug die PO-Desorptionsrate >95 %.

### Beispiel 3

Das Reaktionsgas (Analyse am Reaktorausgang; vor Adsorber) enthält 1,5 Vol.-% Propenoxid, 2,5 Vol.-% Wasser, 0,15 Vol.-% Nebenprodukte (u.a. Acetaldehyd, Propionaldehyd, Aceton, Essigsäure). Das Reaktionsgas wurde durch einen Adsorber geleitet, der mit 5 g Aktivkohle (Firma Degussa) gefüllt wurde. Die Propenoxid-Gaskonzentration nach dem Adsorber wurde per GC in Abhängigkeit der Zeit gemessen.

Die Kapazität von Aktivkohle an Propenoxid beträgt ca. 200 g Propenoxid/(kg Aktivkohle x h).

| Zeit [h] | Propenoxid-Konzentration in der Gasphase [Vol.-%] nach dem Absorber |
|---|---|
| 0 | 0 |
| 1 | 0 |
| 2 | 0 |
| 2,5 | 0 |
| 3 | 0 |
| 4 | 0,5 |
| 5 | 1,4 |
| 6 | 1,45 |
| 7 | 1,45 |

Das adsorbierte Epoxid kann unter Druckverminderung auf 100 mbar zu 95 % desorbiert werden. Es wurden fünf Zyklen "Beladen des Adsorbens" und "Regeneration des Adsorbens" gefahren. Ab Zyklus 2 betrug die PO-Desorptionsrate >95 %.

## Patentansprüche

1. Verfahren zur katalytischen partiellen Oxidation von Kohlenwasserstoffen in Gegenwart von Sauerstoff und wenigstens einem Reduktionsmittel, **dadurch gekennzeichnet, dass** man das Reaktionsgemisch durch eine Katalysator enthaltende Schicht und eine nachgeschaltete Adsorptionsmittel enthaltende Schicht, in der der partiell oxidierte Kohlenwasserstoff adsorbiert wird, leitet.

2. Verfahren nach Anspruch 1, **dadurch gekennzeichnet, dass** man nach der Adsorption des partiell oxidierten Kohlenwasserstoffes das Reaktionsgas in die Reaktion zurückführt.

3. Verfahren nach Anspruch 1 oder 2, **dadurch gekennzeichnet, dass** man die Adsorption des partiell oxidierten Kohlenwasserstoffes in Gegenwart von nicht-kondensierbaren Gasen durchführt.

4. Verfahren nach einem der Ansprüche 1 bis 3, **dadurch gekennzeichnet, dass** man als Adsorptionsmittel Zeolithe und/oder Aktivkohlen einsetzt.

5. Verfahren nach einem der Ansprüche 1 bis 4, **dadurch gekennzeichnet dass** man als Adsorptionsmittel hydrophobe und/oder durch Silylierung und/oder Behandlung mit fluororganischen Verbindungen organisch modifizierte Zeolithe einsetzt.

6. Verfahren nach einem der Ansprüche 1 bis 5, **dadurch gekennzeichnet, dass** man das Adsorptionsmittel nach der Adsorption des partiell oxidierten Kohlenwasserstoffes mittels Desorption regeneriert.

7. Verfahren nach Anspruch 6, bei dem man die Desorption mittels Druckwechseldesorption, Temperaturwechseldesorption oder durch Behandlung mit Wasserdampf durchführt.
